# EUROPEAN PATENT APPLICATION

(11) **EP 2 055 317 A2**
(43) Date of publication of application: **06.05.2009**
(21) Application number: 08270002.2
(22) Date of filing: 19.06.2008
(51) Int. Cl.: A61L 2/00, C02F 1/32

(54) **Fluid disinfection apparatus and method**

(30) Priority: 19.06.2007 GB 0711746; 31.01.2008 GB 0801767
(71) Applicant: GB Environmental Limited, Chelmsford, Essex CM1 3AE (GB)
(72) Inventor: Snowballl, Malcolm Robert, Epping, Essex CM16 6TW (GB)
(74) Representative: Evans, Huw David Duncan

(57) **Abstract**

An apparatus for disinfecting fluids comprises a thin flow channel 12 which extends between a pair of parallel discs 16,18. The fluid to be treated flows radially of the discs 16,18 through the flow channel 12 where it is irradiated axially of the discs by ultra-violet light emitted by one or more lamps 14. At least one of the discs 16,18 is rotated to cause relative movement therebetween. The relative rotation of the discs 16,18 creates a turbulent flow of fluid within the flow channel 12 to ensure that the fluid circulates and swirls close to the opposed surfaces of the discs, thereby ensuring that all parts of the fluid are exposed to an intense dose of ultra-violet radiation. The thin planar flow channel 12 enables a relatively large volume of fluid to be efficiently disinfected since the ultra-violet radiation can pass axially of the discs 16,18 through the thin fluid layer over the large surface area of the discs 16,18 without suffering from significant UV absorption.

## Description

The present invention relates to disinfection apparatus and a method for disinfecting a fluid.

It is well known that high intensity ultra-violet (UV) light has germicidal properties that can be used to disinfect liquids; EP0202891 discloses a device which utilises these properties. The germicidal effect of UV light occurs when the UV light is transmitted through the liquid and results in the disruption of the DNA of any micro-organisms within the liquid, thereby killing or inactivating the micro-organisms.

However, the germicidal effect of UV light is dependent on the UV light passing through the water being disinfected. This can be particularly difficult if the water is viscous or contains a lot of waste material, since the UV light can become totally absorbed after penetrating only a short distance. In these circumstances, a large amount of energy is required to produce the high intensity UV light that is required to penetrate the water.

Drinks and food producers often pack and sell liquids which have transmission coefficients as low as 2% through only 2mm of liquid (i.e. 98% of the incident light is absorbed in passing through 2mm of the liquid). Consequently, known UV light disinfection techniques, such as that disclosed in EP0202891 cannot satisfactorily disinfect these types of liquids. As a result, low transmission coefficient liquids such as fruit juice, cordials, ketchup, soup and post mix beverage syrups have to be thermally pasteurised for disinfection. This process requires a large amount of energy and results in a degradation in the taste of the liquid.

The accepted view therefore is that UV disinfection is not practical for liquids having such a low transmission coefficient for UV light, since it would require the use of extremely high intense UV light combined with a very low processing rate.

We have now devised an apparatus and method which overcomes these problems.

According to a first aspect of the invention there is provided a fluid disinfection apparatus, said apparatus comprising an inlet for receiving fluid to be disinfected into said apparatus, a first member and a second member arranged at a spaced apart location to define a flow channel therebetween, said flow channel being arranged between said inlet and an outlet of the apparatus, said apparatus further comprising irradiation means for irradiating fluid in said flow channel, wherein said first member rotates relative to said second member to agitate the fluid.

The intensity of the radiation decreases as it passes through the fluid. However, the relative rotation of the members creates a turbulent flow of fluid within the apparatus to ensure that the fluid circulates and swirls close to the opposed surfaces of the members, thereby ensuring that all parts of the fluid are exposed to an intense dose of radiation. In this manner it is found that the disinfection rate increases with an increase in the relative rotation of the members, since this increases the agitation of the fluid.

Preferably said flow channel comprises a substantially planar space between said first and second members.

Preferably said first member rotates relative to said second member about a rotational axis, the flow channel being arranged such that fluid flows radially of the axis of rotation.

Preferably, the irradiation means emits radiation substantially normal to the planar flow channel.

In one embodiment, the second member is fixed and the first member rotates. In an alternative embodiment the first and second members each rotate with different speeds and/or in opposite directions.

Preferably the apparatus comprises a third member arranged at a spaced apart location to said first member to define a further flow channel therebetween, said further flow channel being in fluid communication with said inlet, said irradiation means being arranged to irradiate fluid in said further flow channel, wherein said first member also rotates relative to said third member to agitate the fluid.

The further flow channel may be arranged in series or parallel with said first-mentioned flow channel.

Preferably the opposed surfaces of the members extend generally parallel to each other, said first member being arranged to rotate relative to said third member to agitate the fluid.

The or each rotatable member is preferably flat and preferably disc shaped. Preferably the opposed surface of the or each member is formed from a material which is substantially transmissive to said radiation, said irradiation means irradiating fluid in said flow channel(s) through the or each said surface.

The turbulent flow further creates a scrubbing action upon the surfaces of the members which prevents the build up of material on said surfaces which may reduce the transmission of the radiation into the fluid, and thus the disinfecting ability. Preferably the surface of at least one of the members is profiled to promote mixing.

The first member is preferably mounted upon a rotatable shaft, such that said first member extends substantially radially therefrom.

Preferably, a central duct extends from a point radially inwardly of the first member to enable fluid to pass into or out of the flow channel formed between the first and second members. Said central duct is preferably formed within the rotatable shaft.

Preferably, a radial duct extends from a point radially outwardly of the first member to enable fluid to pass out of or into the flow channel formed between the first and second members.

In one embodiment the central and radial ducts enable fluid to pass into or out of the flow channel formed between the first and third members in parallel with the flow channel formed between the first and second members.

In an alternative embodiment, the central and radial ducts enable fluid to pass into or out of the flow channel formed between the first and third members in series with the flow channel formed between the first and second members.

Preferably, the spacing between the members is adjustable axially of the direction of rotation of the first member. Preferably, the fluid passes into the apparatus under a controlled pressure. By controlling the pressure at the inlet and maintaining a consistent separation of the members, a controlled flow of fluid can be maintained through the apparatus, independently of the viscosity of the fluid.

The fluid pressure is preferably controlled by a pump and/or a pressure regulator.

Preferably, the irradiation means comprises at least one UV light source for disinfecting the fluid. The or each light source preferably comprises at least one UV lamp.

The or each UV light source preferably comprises a series of annular UV lamps concentrically arranged about the axis of rotation. Alternatively, the or each UV light source may comprise a coiled UV lamps arranged about the axis of rotation. In any of the UV lamp alternatives, however, it is preferred that the UV light generated by the at least one UV light source comprises one or more wavelengths in the range 220nm - 280nm, said wavelengths being germicidal wavelengths.

Preferably, the at least one UV light source is movably mounted within a lighting assembly to enable selective positioning of the at least one light source with respect to the members. The lighting assembly preferably comprises a mirrored reflector extending partially around the or each light source, for directing the UV light onto the fluid.

The lighting assembly is preferably housed within an UV non-transmitting material to ensure that the UV light emitted by the or each UV light source is retained within the housing. Preferably, said housing comprises an inner mirrored surface.

The members preferably permit the transmission of UV light from the or each UV light source, to permit disinfection of the fluid passing there between. Preferably, the members are quartz discs.

According to a second aspect of the invention, there is provided a method of disinfecting fluid, said method comprising passing a fluid to be disinfected from an inlet to an outlet through a flow passage defined between a first member and a second member, said method further comprising rotating the first member relative to the second member to agitate the fluid therebetween and irradiating the fluid in the flow passage with irradiation means.

The present invention will now be described by way of example only and with reference to the accompanying drawings, in which:
Figure 1 is a sectional view of an embodiment of fluid disinfection apparatus in accordance with this invention; and
Figure 2 is a sectional view of an alternative embodiment of fluid disinfection apparatus in accordance with this invention.

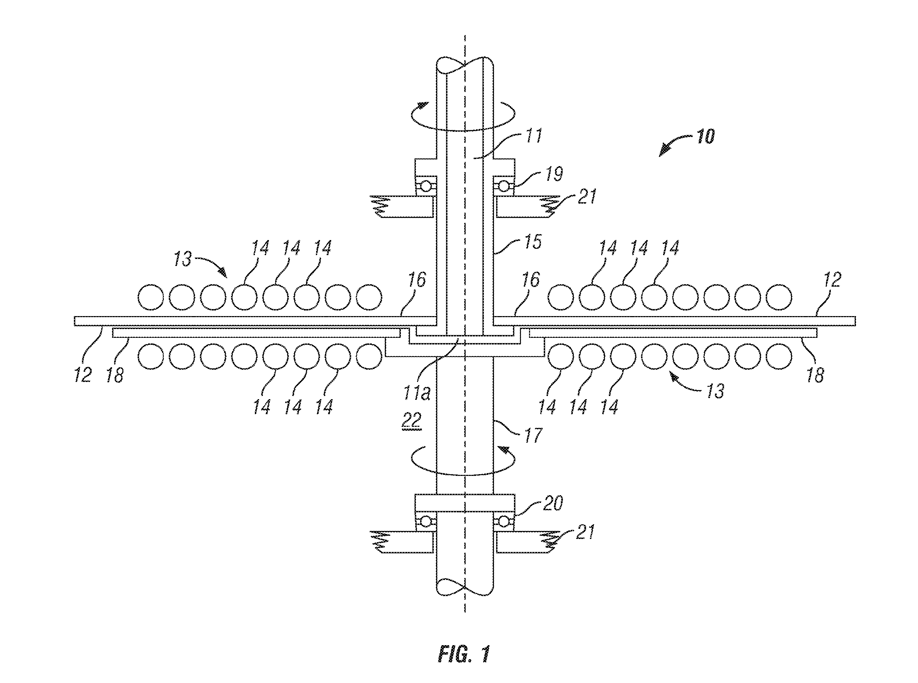

Referring to Figure 1 of the drawings, there is shown a fluid disinfection apparatus 10 comprising an central duct 11 for receiving fluid to be disinfected into the apparatus from an inlet thereof. The inlet 11 is formed as a bore of a first drive shaft 15 and is in communication with a pressurised source of fluid to be disinfected. The fluid pressure is controlled by a pressure regulator (not shown); by controlling the pressure of the fluid, a controlled rate of fluid flow can be maintained through the apparatus.

The distal end of the first drive shaft is secured to the central portion of a first disc 16, with the bore 11 extending through the disc. Adjacent the first drive shaft 15 there is arranged a second drive shaft 17, the distal end of which is secured to the central portion of a second disc 18.

The first and second drive shafts 15,17 are arranged co-axially with each other. The drive shafts are mounted to bearing sleeves 19 and 20, which permit the drive shafts 15,17 to be rotated about their longitudinal axis. Adjustment means such as a translation stage (not shown) secured to the proximal end of one or both shafts 15, 17 enable the discs to be brought together or separated by sliding the rotatable shafts within the sleeves 19, 20. A planar flow channel 12 is defined between the discs 16,18.

The proximal end of each drive shaft 15, 17, is further mounted to drive means such as a motor (not shown), to permit the shafts and thus the discs 16, 18 secured thereto to selectively rotate at a given speed and/or in a given direction. Surrounding the discs 16, 18 at either side of the planar flow channel 12, there is provided a UV lighting assembly 13 comprising a plurality of UV tubes 14 partially housed between mirrored reflectors (not shown) which act to direct the UV light onto the discs16, 18. The tubes 14 may be of the linear type or circular and furthermore in the latter case, the UV tubes may be concentrically arranged around the drive shafts 15, 17.

The UV lighting assembly 13 and discs 16, 18 are housed with a housing 21 which prevents UV light escaping the disinfection chamber 22 formed therein and which itself may be formed with mirrored inner surfaces to help concentrate the UV light upon the discs and thus the fluid therebetween.

In use, fluid to be disinfected is passed through the bore 11 of the first drive shaft 15 under pressure and enters the planar flow channel 12 via port 11 a of the bore 11. The thin planar flow channel 12 enables a relatively large volume of fluid to be efficiently disinfected since the UV light generated by the UV lamp assembly can extend axially of the discs through the thin fluid layer over the large surface area of the discs 16,18 without suffering from significant UV absorption. Fluid disinfected by the UV lamp assembly 13 then passes out from between the discs 16,18 to the outlet (not shown).

To further enhance the disinfection, the drive shafts and thus the discs 16,18 are caused to rotate with different speeds and/or in opposite directions by the motor (not shown). The relative rotation of the discs establishes a turbulent flow within the planar flow channel 12 and ensures that all parts of the fluid circulate close to the surface of the discs 16,18 and receive an intense dose of UV radiation and therefore become effectively disinfected.

As an additional advantage, the turbulent flow acts to scrub the opposed surfaces of the discs 16,18, thereby preventing the build-up of scum or slime on the discs. This is beneficial since such dirt would absorb the UV light and effectively reduce the disinfecting ability of the system.

The rotation of the discs 16,18 causes the fluid to pass out from the flow channel 12 under the influence of the centrifugal force, however, the rate at which the fluid passes out from the flow channel 12 has been found difficult to control by relying on the rotation of the discs. Moreover, for the situation in which the discs rotate in opposite directions, there is only a weak resultant force acting upon the fluid in the radial direction. Accordingly, better control of the fluid flow rate is achieved by controlling the pressure at the inlet.

The amount of fluid passing through the flow channel 12 can be increased by increasing the separation of the discs 16, 18. However, presenting and maintaining a very thin layer of fluid to the UV radiation, enables the UV light to pass completely through the fluid without suffering from the significant absorption associated with a thick layer. Accordingly, to increase the fluid output from the apparatus while maintaining the correct disinfection, it is found more beneficial to increase the inlet pressure coupled with an increase in the relative rotation of the discs 16, 18. The increased relative rotation creates a more turbulent fluid flow between the discs 16, 18 thereby ensuring that all parts of the fluid pass close to the surface of the discs 16, 18 more readily and frequently (and therefore receive an intense dose of UV radiation for disinfection) to accommodate the increased flow rate.

In an alternative embodiment it is envisaged that fluid could also pass in the opposite direction, i.e. into outlet 12 and out through inlet 11, and still produce the same beneficial disinfection. In this case however, it may be necessary to apply a controlled suction to the inlet 11 to regulate the flow of fluid.

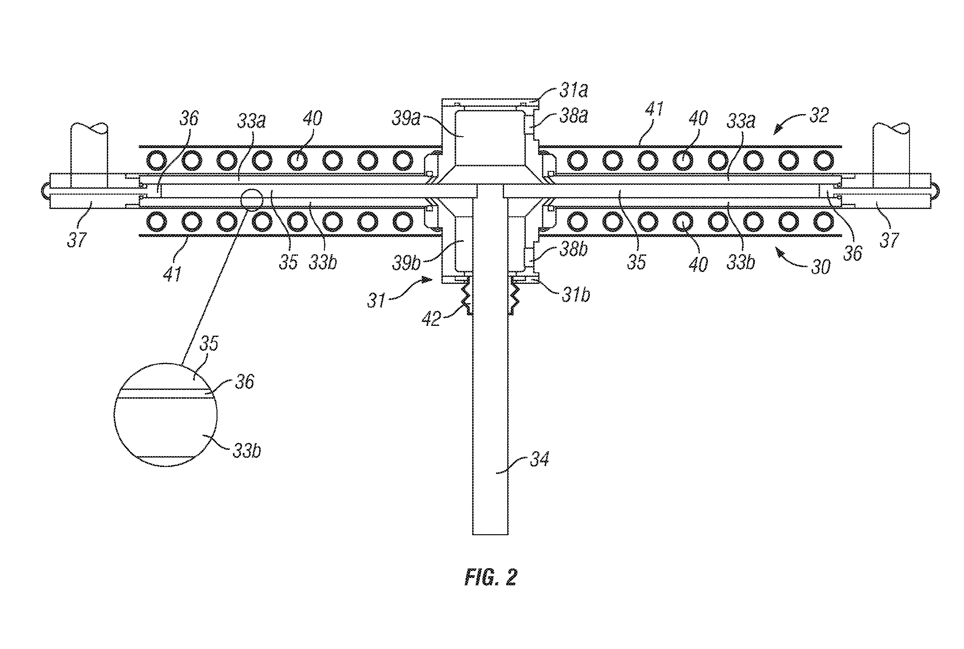

Referring to Figure 2 of the drawings, there is shown an alternative embodiment of a fluid disinfection apparatus comprising a circular body 30 having a central hub portion 31 and a radially outer portion 32. The hub portion 31 comprises two spatially-separated halves 31a,31b which are mounted co-axially of each other. The outer portion 32 comprises pair of parallel quartz plates 33a,33b which extend from the respective halves 31 a,31 b of the hub 31. An elongate shaft 34 extends axially through one of the halves 31 b of the hub 31. A disc 35 is mounted to the inner end of the shaft 34 and extends normal thereto in an annular space 36 defined between the plates 33a,33b. The radially outer edges of the plates 33a,33b are sealed against an annular outlet manifold 37 of the body 30.

Each of the halves 31 a,31 b over the hub 31 comprise radially extending inlet ports 38a,38b, which connect to respective chambers 39a,39b formed inside the hub 31. The chambers 39a,39b lead to respective portions of the annular space 36 on opposite sides of the disc 35.

A plurality of concentrically-mounted annular lamps 40 are disposed behind each plate 33a, 33b and are arranged to direct ultra-violet light through the plates into the space 36. The lamps 40 may alternatively comprise curved or coiled lamps. A reflector 41 is disposed behind each lamp 40 to reflect the emitted light towards the space 36. Each major surface of the disc 35 may be reflective and is preferably patterned with a spiral formation extending in an opposite sense to the direction of rotation of the shaft 34.

The halves 31 a,31 b of the hub 31 and the plates 33a,33b can be moved axially towards and away from the disc 35, in order to vary the width of the space 36 in which the disc 35 is mounted. A seal 41 on the hub half 31 b allows the hub half to move axially relative to the shaft 34 and also provides a rotational seal.

In use, the shaft 34 is rotated inside the space 36 whilst fluid flows from the inlets 38a,38b to the outlet 37 through the space 36 on opposite sides of the disc 35. It will be appreciated that the fluid does flow as a thin film over the opposite sides of the disc 35 where it is irradiated by the ultra-violet light emitted by the lamps 40. The rotation of the disc 35 inside the space 36 agitates the laminar flow and causes turbulence therein. This effect is enhanced by the provision of the spiral formations on the disc 35.

In an alternative embodiment, the ports 38a,38b on hub 31 may respectively form inlet and outlet ports of the apparatus, such that the fluid flows over one surface of the disc 35 then returns via the opposite surface.

From the foregoing therefore it is evident that the system provides an efficient method of disinfecting fluids and particularly fluids having a low UV transmission, by presenting a very thin turbulent layer of fluid to the UV light.

## Claims

1. Fluid disinfection apparatus comprising an inlet for receiving fluid to be disinfected into said apparatus, a first member and a second member arranged at a spaced apart location to define a flow channel therebetween, said flow channel being arranged between said inlet and outlet of the apparatus, said apparatus further comprising irradiation means for irradiating fluid in said flow channel, wherein said first member rotates relative to said second member to agitate the fluid.

2. Fluid disinfection apparatus as claimed in claim 1, in which said flow channel comprises a substantially planar space between said first and second members.

3. Fluid disinfection apparatus as claimed in claim 2, in which said first member rotates relative to said second member about a rotational axis, the flow channel being arranged such that fluid flows radially of the axis of rotation.

4. Fluid disinfection apparatus as claimed in claim 3, in which the second member is fixed and the first member rotates.

5. Fluid disinfection apparatus as claimed in claim 3, in which the first and second members each rotate at different relative speeds and/or in opposite directions.

6. Fluid disinfection apparatus as claimed in claim 3, in which the apparatus comprises a third member arranged at a spaced apart location to said first member to define a further flow channel therebetween, said further flow channel being in fluid communication with said inlet, said irradiation means being arranged to irradiate fluid in said further flow channel, wherein said first member also rotates relative to said third member to agitate the fluid.

7. Fluid disinfection apparatus as claimed in any preceding claim, in which the or each rotatable member is flat and disc shaped.

8. Fluid disinfection apparatus as claimed in any preceding claim, in which the opposed surface of the or each member is formed from a material which is substantially transmissive to said radiation, said irradiation means irradiating fluid in said flow channel(s) through the or each said surface.

9. Fluid disinfection apparatus as claimed in any preceding claim, in which a central duct extends from a point radially inwardly of the first member to enable fluid to pass into or out of the flow channel formed between the first and second members.

10. Fluid disinfection apparatus as claimed in claim 9, in which said central duct is formed within a shaft to which the first member is mounted.

11. Fluid disinfection apparatus as claimed in any preceding claim, in which a radial duct extends from a point radially outwardly of the first member to enable fluid to pass out of or into the flow channel formed between the first and second members.

12. Fluid disinfection apparatus as claimed in claim 11 as appended to claim 9, in which the central and radial ducts enable fluid to pass into or out of the flow channel formed between the first and third members in parallel with the flow channel formed between the first and second members.

13. Fluid disinfection apparatus as claimed in claim 11 as appended to claim 9, in which the central and radial ducts enable fluid to pass into or out of the flow channel formed between the first and third members in series with the flow channel formed between the first and second members.

14. Fluid disinfection apparatus as claimed in any preceding claim, in which the spacing between the members is adjustable axially of the direction of rotation of the first member.

15. A method of disinfecting fluid, said method comprising passing a fluid to be disinfected from an inlet to an outlet through a flow passage defined between a first member and a second member, said method further comprising rotating the first member relative to the second member to agitate the fluid therebetween and irradiating the fluid in the flow passage with irradiation means.
